# FASCICULE DE BREVET EUROPEEN

(11) **EP 0 443 894 B1**
(45) Date de publication et mention de la délivrance du brevet: **09.08.1995**
(21) Numéro de dépôt: 91400240.7
(22) Date de dépôt: 31.01.1991
(51) Int. Cl.: A61B 17/58

(54) **Dispositif d'ostéosynthèse pour la correction des déviations rachidiennes**
Osteosynthesevorrichtung zur Berichtigung der Verformungen der Wirbelsäule
Osteosynthesis device for correction of spinal deformation

(30) Priorité: 19.02.1990 FR 9001970
(43) Date de publication de la demande: 28.08.1991
(73) Titulaire: SOCIETE DE FABRICATION DE MATERIEL ORTHOPEDIQUE SOFAMOR, 75016 Paris (FR)
(72) Inventeur: Dubousset,Jean, F-92140 Clamart (FR)
(74) Mandataire: Martin, Jean-Paul

(56) Documents cités:
- EP-A- 0 128 058
- EP-A- 0 348 272
- US-A- 4 289 123
- US-A- 4 773 402

## Description

La présente invention a pour objet une plaque pour dispositif d'ostéosynthèse de correction des déviations rachidiennes. US-A-4 289 123 décrit un dispositif d'ostéosynthèse.

On sait que l'abord antérieur du rachis dorso-lombaire trouve des applications dans les cas suivants :
- En traumatologie, dans le cas d'une fracture comminutive. Cette instabilité maximum du rachis oblige en effet à un soutien antérieur.
- En neuro-chirurgie : l'ablation du corps vertébral, consécutive à une tumeur osseuse, oblige à associer à la greffe un système d'étaiement.
- En déviations rachidiennes : certaines déviations avec forte rotation, surtout au niveau lombaire, exigent une approche antérieure pour restituer une meilleure lordose. Il s'agit alors, soit de corriger et maintenir une scoliose, soit de traiter une cyphose. Dans ce dernier cas, la cyphose peut être à grand rayon de courbure et harmonieuse, ou au contraire très courte (cyphose angulaire).

Pour ces cyphoses courtes, plusieurs étio-logies sont à distinguer, parmi lesquelles peuvent être essentiellement retenues les malformations congénitales (hémivertèbres), les infections (mal de Pott), les traumatismes récents (fractures comminutives du corps vertébral) ou anciens, et les tumeurs bénignes ou surtout malignes (métastases). Dans le cas d'une tumeur atteignant le corps vertébral, même en l'absence de cyphose, l'abord antérieur est justifié.

Parmi les réalisations connues, on distingue deux types de dispositifs permettant l'abord antérieur:
a) les systèmes permettant une distraction, ou une compression par des tiges filetées et des boulons,
b) les plaques.

Les systèmes en distraction sont dérivés d'une instrumentation connue, utilisée dans le traitement des scolioses. Leur principe est constant : une ou deux vis sont fixées sur le corps vertébral des vertèbres, et elles sont reliées par une ou deux tiges permettant de réaliser une distraction, c'est-à-dire une extension des vertèbres, et ainsi de corriger la cyphose. Puis une fois ces tiges fixées aux vis, on stabilise le montage entre les corps vertébraux. Ces systèmes présentent l'avantage de permettre une distraction ou une compression, mais ne sont pas assez stables avec une seule tige, ou trop volumineux avec deux tiges. Certaines de ces réalisations connues ne permettent pas non plus de dérotation, présentent des risques vasculaires ainsi que de pseudoarthroses. On constate en outre également des pertes angulaires.

Les plaques sont fixées aux corps vertébraux par deux ou trois vis. Elles présentent l'avantage d'être moins saillantes et habituellement plus solides que les systèmes par tiges. En revanche leur utilisation ne permet pas de pratiquer une distraction directe, et donc une correction instrumentale de la cyphose ou de la scoliose.

L'invention a donc pour but de réaliser un dispositif d'ostéosynthèse par voie antérieure qui présente, une fois mis en place, des saillies minimales par rapport aux vertèbres, surtout antérieurement pour éviter tout contact avec les vaisseaux tels l'aorte et la veine cave, qui permette une correction effective des déviations rachidiennes avec une bonne tenue mécanique dans le temps et sous charges répétées, et enfin dont la mise en place par le chirurgien soit la plus simple possible.

La plaque pour dispositif d'ostéosynthèse selon l'invention comprend les moyens mentionnés à la revendication 1.

Suivant une particularité de l'invention, au moins l'alésage le plus antérieur de la plaque, est chanfreiné pour recevoir une vis pourvue d'une tête conique épousant le chanfrein, afin de permettre une orientation de la vis dans le corps vertébral et d'éviter toute saillie de la tête de la vis.

Les deux alésages ont une orientation caudale ou crâniale, et sont décalés dans un plan sagittal.

Suivant une autre caractéristique de l'invention, les alésages sont ménagés au voisinage des extrémités de la plaque et les moyens de solidarisation de la plaque et de la tige comprennent un trou taraudé reliant la surface extérieure cintrée au trou du bossage, et une vis adaptée pour être vissée dans ce trou taraudé.

Le dispositif d'ostéosynthèse conforme à l'invention comprend en combinaison :
- Deux tiges destinées à être fixées respectivement sur une partie antérieure et sur une partie latérale ou postérieure des corps vertébraux,
- Des moyens de fixation de la tige latérale ou postérieure aux vertèbres,
- Des plaques dont les bossages peuvent être traversés par les extrémités opposées de la tige antérieure,
- Des moyens de blocage d'au moins la tige antérieure dans les plaques et des moyens d'ancrage des plaques dans les corps vertébraux des vertèbres associées.

Suivant d'autres caractéristiques de l'invention, le dispositif comprend des moyens de liaison transversale rigide des deux tiges entre elles, constituant avec ces dernières un montage en cadre rigide.
- Les moyens de liaison transversale rigide des tiges sont des barres rigides équipées chacune de deux crochets terminaux délimitant des gorges de réception des tiges, ainsi que d'organes de blocage des tiges dans les gorges des crochets,
- Les moyens de fixation de la tige latérale ou postérieure sont des vis du type comportant un corps en U et un bouchon fileté de blocage de la tige dans le corps, pouvant se visser à l'intérieur du corps en U sur des taraudages de ses branches latérales.

Ce dispositif d'ostéosynthèse pour correction antérieure est indiqué tout particulièrement pour les scolioses lombaires, thoraco-lombaires et thoraciques. Il peut concerner plusieurs niveaux. Les tiges sont de préférence du type à aspérités, la tige latérale ou postérieure est cintrée, et par dérotation, fournit la correction tridimensionnelle du segment rachidien instrumenté.

L'invention sera maintenant décrite en référence aux dessins annexés qui en illustrent plusieurs formes de réalisation à titre d'exemples non limitatifs.

La figure 1 est une vue en élévation latérale d'un segment rachidien muni d'un dispositif d'osthéosynthèse conforme à l'invention.

La figure 2 est une vue en perspective à échelle agrandie d'une des plaques du dispositif d'ostéosynthèse de la Fig.1.

La figure 3 est une vue en perspective, sous un angle différent, de la plaque de la Fig.2 et de deux vis équipant celle-ci.

La figure 4 est une vue en perspective de la plaque des Fig.2 et 3 munie des deux vis et traversée par une tige antérieure bloquée par une troisième vis équipant la plaque.

La figure 5 est une vue en perspective à échelle agrandie d'une des vis d'ancrage osseux de la tige latérale ou postérieure du dispositif de la Fig.1.

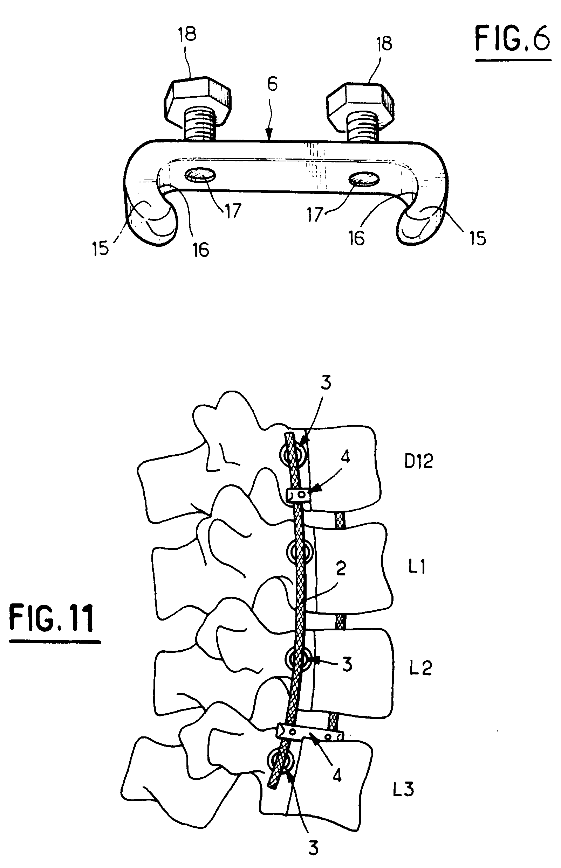

La figure 6 est une vue en perspective d'un des éléments de liaison transversale rigide des deux tiges du dispositif de la Fig.1.

Les figures 7 à 11 sont des vues en élévation illustrant la séquence opératoire de mise en place par un chirurgien du dispositif de correction des Fig.1 à 6 sur un segment rachidien correspondant.

La figure 12 est une vue en perspective d'un second mode de réalisation de la plaque du dispositif de la Fig.1 et des vis équipant cette plaque.

La figure 13 est une vue en perspective d'une variante de réalisation de la plaque de la Fig.11.

La figure 14 est une vue en élévation d'un dispositif d'ostéosynthèse selon l'invention, comportant plusieurs plaques selon les Fig.11 et 12.

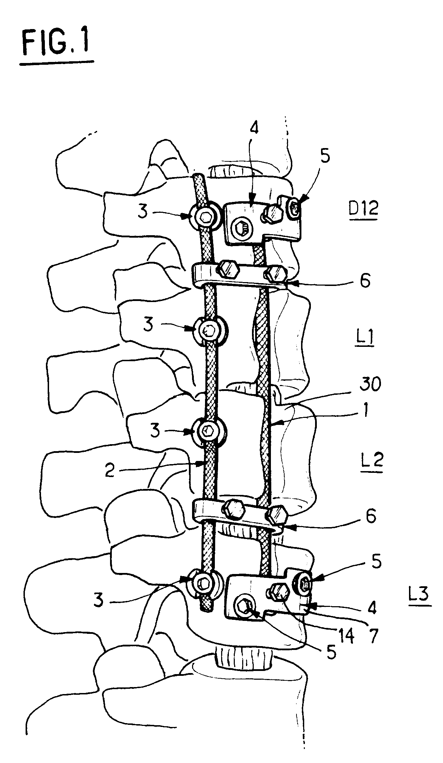

Le dispositif d'ostéosynthèse représenté à la Fig.1 est destiné à la correction des déviations rachidiennes d'un segment dorso-lombaire comprenant par exemple quatre vertèbres D12, L1, L2, L3.

Il comprend les éléments suivants :
- Deux tiges 1, 2 destinées à être fixées respectivement sur une partie antérieure et sur une partie latérale ou postérieure des corps vertébraux,
- Des moyens de fixation de la tige latérale ou postérieure 2 aux vertèbres, constitués dans l'exemple représenté par des vis 3 d'ancrage osseux au nombre de quatre, réparties d'une extrémité à l'autre de la tige 2,
- Deux plaques 4 agencées pour permettre la fixation de la tige antérieure 1 aux corps vertébraux des vertèbres D12-L3,
- Des moyens de blocage dans les plaques 4 de la tige antérieure 1, ainsi que des moyens d'ancrage des plaques 4 dans les corps vertébraux, constitués dans l'exemple décrit par deux vis 5 à os spongieux traversant chaque plaque 4,
- Et des organes 6 de liaison transversale rigide des deux tiges 1, 2 entre elles, constituant avec ces dernières un montage en cadre rigide.

Les tiges 1 et 2 sont de préférence moletées sur toute leur longueur, et réalisées par exemple en acier renforcé. Elles peuvent être de tailles différentes suivant le nombre de niveaux instrumentés, leurs diamètres pouvant être par exemple de 4mm. Lors d'un abord antérieur du segment rachidien, l'une des deux tiges 1, 2 a pour fonction de corriger le segment (tige postérieure 2), et l'autre (tige antérieure 1), stabilise et donne toute sa rigidité au montage.

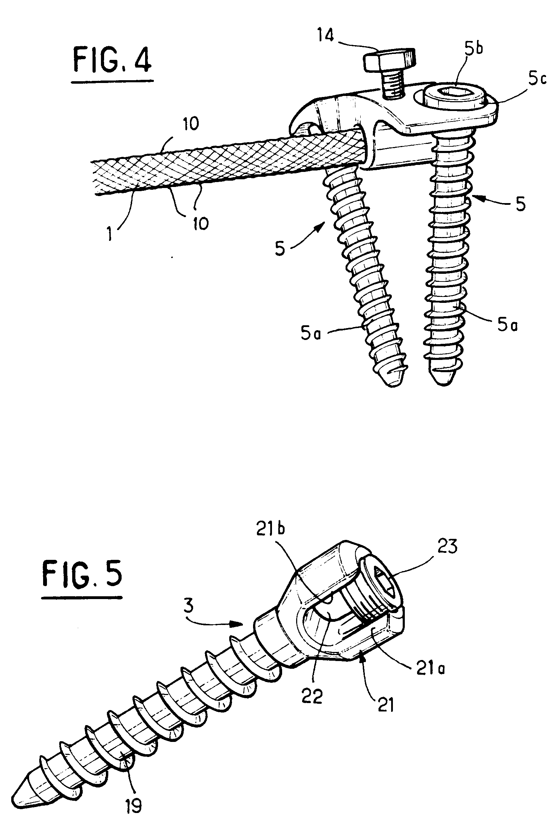

Chaque plaque 4 est incurvée et présente une surface extérieure cintrée 7 correspondant à l'anatomie antéro-latérale des corps vertébraux. Sur sa face intérieure, opposée à la surface extérieure 7, elle comporte un bossage 8 percé d'un trou 9 de passage de la tige antérieure 1. Deux alésages 11, 12 sont de plus percés dans chaque plaque 4 au voisinage de ses extrémités, ces alésages étant adaptés pour recevoir chacun une vis 5, du type "à os spongieux", constituée d'une tige filetée 5a pour os spongieux et d'une tête 5b (Fig.4) creusée d'un trou hexagonal permettant le serrage de la vis. Au moins l'alésage 12 le plus antérieur présente un chanfrein pouvant coopérer avec une surface conique 5c de la tête 5b, ce qui permet d'orienter la vis 5 dans le corps vertébral de la vertèbre (D12, L3). Le second alésage 11 peut également présenter un chanfrein 11a similaire au chanfrein conique 12a.

Ces deux alésages 11, 12 peuvent comporter une orientation par exemple caudale s'ils concernent la vertèbre sous-jacente, et par exemple crâniale s'ils concernent la vertèbre sus-jacente. Ils sont décalés dans un plan sagittal, afin d'éviter toute interférence entre les deux vis 5 de fixation et pour augmenter la stabilité de l'ancrage.

Un trou taraudé 13 est ménagé dans chaque plaque 4 perpendiculairement à l'axe du trou traversant 9 et relie la surface cintrée 7 au trou 9. Une vis 14 (Fig.4) peut être vissée dans le trou 13 pour bloquer la tige 1 en translation et en rotation par rapport à la plaque 4.

Les organes rigides 6 de liaison des tiges 1 et 2, sont au nombre de deux : chaque organe 6 est constitué par une barre équipée de deux crochets terminaux 15 délimitant des gorges 16 de réception des tiges correspondantes 1 et 2. De plus dans chaque barre 6 sont formés, au voisinage des crochets 16, deux trous filetés 17 dans lesquels peuvent venir se visser les tiges filetées de vis 18 à têtes hexagonales, ces vis 18 permettant de bloquer les tiges 1, 2 dans les gorges 16.

Les barres rigides 6 de liaison transversale peuvent être par exemple du type de celles décrites dans la demande de brevet français 89 04 750 déposée le 11 avril 1989 par la Demanderesse.

Les moyens de fixation de la tige latérale ou postérieure 2 sont, dans l'exemple décrit, des vis 3 d'ancrage osseux, comportant une tige filetée 19 et un corps 21 en U, présentant un canal 22 délimité par les deux branches latérales 21a, 21b du corps 21. La tige moletée 2 peut venir se loger dans le canal 22, où elle est bloquée en translation et en rotation par un bouchon fileté 23. Ce dernier se visse à l'intérieur du corps 21 sur des taraudages de ses branches latérales 21a, 21b, la face du bouchon 23 orientée vers la tige 2 peut être pourvue de moyens d'ancrage, tels qu'une pointe centrale venant s'enfoncer dans la surface de la tige.

La vis 3 est avantageusement conforme à celle décrite dans la demande de brevet français 88 08 538 déposée le 24 juin 1988 pa la Demanderesse.

Les vis 3 sont placées en partie postérieure des corps vertébraux, et assurent par la fixation du bouchon 23, l'immobilisation de la tige postérieure 2 sur les vis 3.

Les deux tiges 1, 2 présentent des crans moletés 10 (Fig.4) pouvant coopérer avec des pointeaux des vis 14 pour bloquer les tiges.

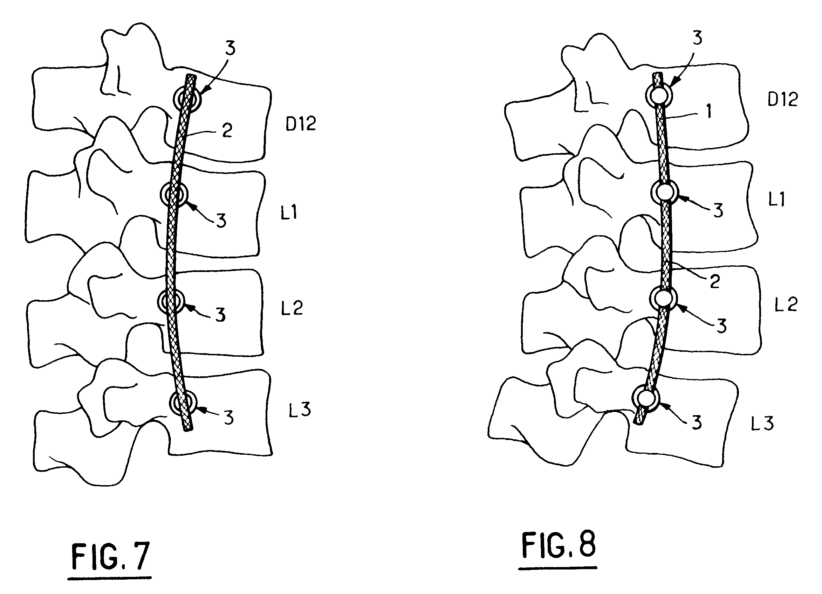

La technique opératoire pour la mise en place du dispositif de correction rachidienne qui vient d'être décrit est la suivante (fig.7 à 11).

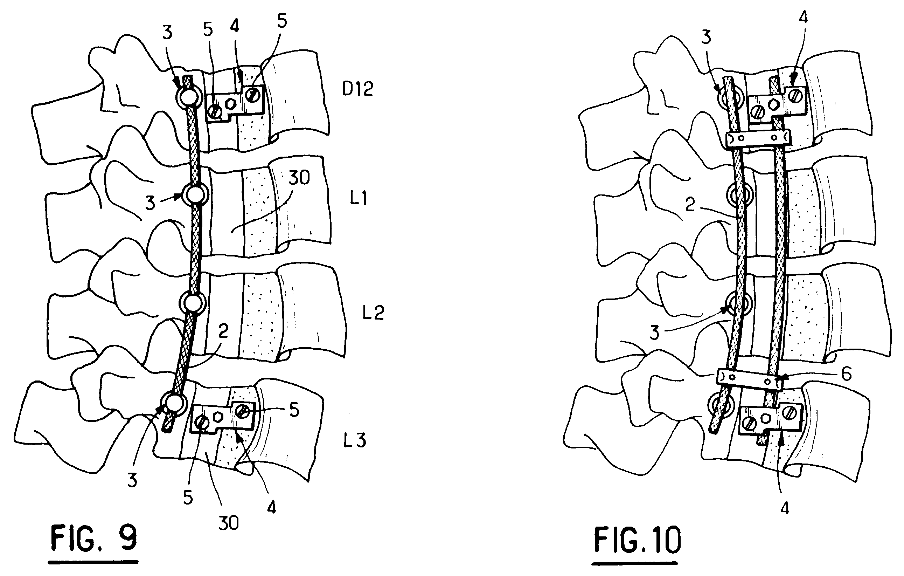

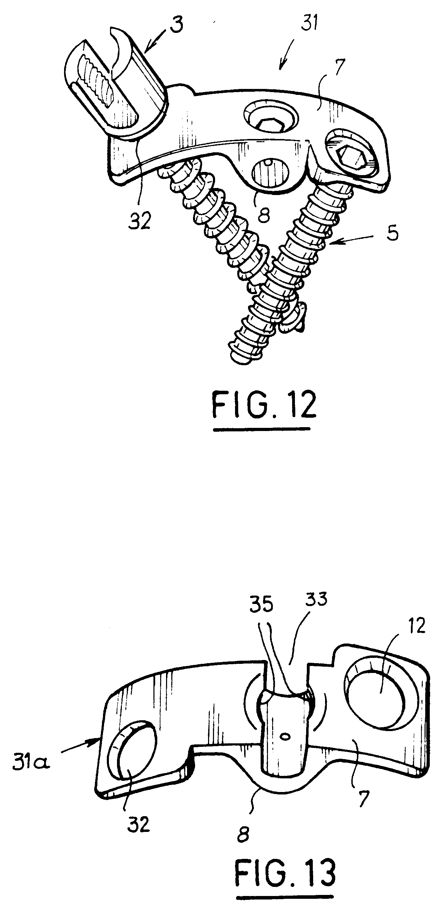

On voit à la Fig.7 un tronçon dorso-lombaire D12-L1-L2-L3, semblable à celui de la Fig.1, et qui est cyphosé :
a) On met en place des vis 3 à bouchons 23 sur la partie postérieure des corps vertébraux.
b) On procède au cintrage de la tige postérieure 2, et on la met en place dans les canaux ou gorges 22 des vis 3 (Fig.7).
c) Le chirurgien exécute une dérotation de la tige 2 et une correction de la cyphose (Fig.8), après laquelle il met en place les bouchons filetés 23 dans les corps 21 des vis 3.
d) Le chirurgien procède à un dépériostage de la partie latérale des corps vertébraux des vertèbres D12 à L3; il réalise ainsi une saignée 30 sur la partie antéro-latérale des corps vertébraux instrumentés (Fig.9).
e) Le chirurgien met en place les deux plaques 4 sur les vertèbres extrêmes D12 et L3 et les fixe dans les corps vertébraux, par insertion dans ceux-ci des deux vis 5 à os (Fig.9).
f) Le chirurgien exécute un cintrage de la tige antérieure 1 et la met en place dans les trous traversants 9 des deux plaques 4 (Fig.10). Il procède ensuite à la distraction des deux plaques 4. Le chirurgien bloque la tige 1 en translation et en rotation dans les plaques 4 grâce aux vis 14, puis la tige 1 est définitivement fixée dans les plaques 4 par rupture des têtes hexagonales des vis 14 (Fig.10).
g) Le chirurgien met en place les barres 6 de fixation transverse et de liaison rigide des tiges antérieure 1 et postérieure 2, qui sont définitivement fixées dans les barres 6 par rupture des têtes des vis 18.
h) Enfin le chirurgien remet en place le périoste pour boucher la saignée 30, le segment rachidien considéré ayant finalement l'aspect représenté à la Fig.11.

En plus des avantages mentionnés précédemment, l'invention présente les suivants :
- Le montage est réalisé en cadre rectangulaire, grâce aux barres rigides de fixation transverses 6, ce qui lui confère une grande solidité et une grande stabilité.
- La mise en place du dispositif de correction est modulaire, élément par élément,
- La tige antérieure 1 est enfouie dans la saignée 30 intérieure aux corps vertébraux, ce qui évite tout contact avec les gros vaisseaux. De même, les chanfreins 11a et 12a des plaques 4 et les têtes coniques complémentaires 5c des vis 5 permettent à ces têtes de s'enfoncer presque entièrement dans les plaques 4.
- Les crans moletés 10 des tiges 1 et 2 assurent une fixation solide de celles-ci aux plaques 4 et aux vis 3.

Dans sa seconde forme de réalisation, illustrée à la Fig.12, la plaque 31 s'étend sur une longueur suffisante pour que son alésage latéral ou postérieur 32 puisse recevoir une vis 3 remplissant alors une double fonction : d'une part une fonction de fixation de la plaque 31 au corps vertébral, et d'autre part une fonction de fixation de la tige latérale ou postérieure 2 à la plaque 31. La vis 3 peut être par exemple du type de celle précédemment décrite (Fig.5) à corps en U 21 et bouchon fileté 23.

L'utilisation des plaques 31 permet donc de supprimer une vis 5 dans le montage de la Fig.1.

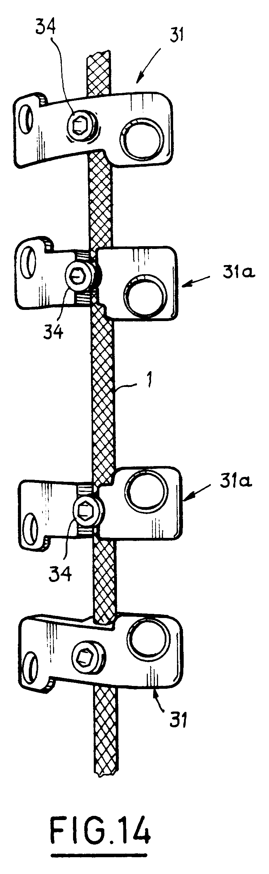

La Fig.13 montre une variante 31a de réalisation de la plaque 31, dont elle diffère par le fait que l'ouverture du bossage 8 est ici un canal 33 longitudinal, ouvert sur la surface extérieure cintrée 7 ainsi interrompue de chaque côté du canal 33 pour y permettre l'insertion de la tige antérieure 1. La plaque 31a est complétée par des moyens de blocage de la tige 1 dans le canal 33, par exemple un boulon 34 (Fig.14) pouvant se visser sur des taraudages 35 usinés sur les rives du canal 33.

La Fig.14 illustre un exemple de réalisation du dispositif d'ostéosynthèse avec des vis 31 et 31a : à ses extrémités le dispositif comprend deux plaques 31, la tige antérieure 1 traversant les bossages 8 et étant bloquée dans ceux-ci par des boulons 34 (ou 14 comme sur la Fig.1), tandis que la tige latérale 2 (non représentée) est fixée par deux vis 3 sur les plaques terminales 31. Le dispositif est complété par des plaques 31a, ici au nombre de deux, disposées entre les plaques 31 pour assurer une liaison transversale rigide des tiges 1, 2 entre elles, la tige antérieure 1 étant bloquée dans les canaux 33 par les boulons 34.

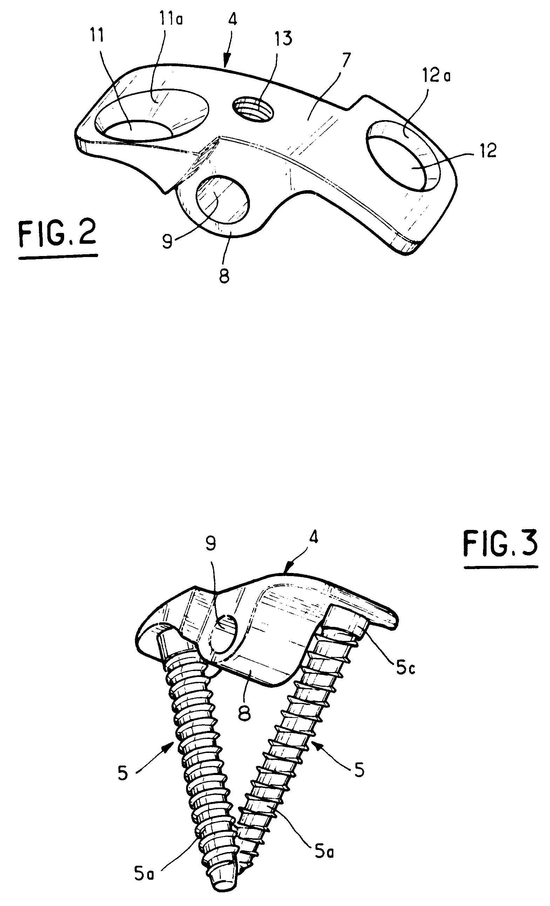

Il convient de noter qu'en variante les plaques intermédiaires 31a peuvent ne pas être du type de celle de la Fig.11 mais du type de celui de la Fig.2. Dans ce cas les plaques intermédiaires n'assurent pas la liaison transversale rigide des tiges entre elles, cette liaison pouvant être assurée par des barres telles que 6.

Enfin les différentes plaques décrites 4, 31, 31a peuvent présenter des picots (non visibles aux dessins) sur leurs faces opposées à la surface extérieure cintrée 7, afin de faciliter l'ancrage de la plaque sur la vertèbre ainsi que la mise en place du matériel par le chirurgien. Il est également possible d'utiliser entre les plaques terminales 4 ou 31, des plaques allongées 31a et des dispositifs de liaison transverse tels que 6. Les tiges 1, 2 peuvent être réalisées conformément au brevet européen 0 128 058, et les organes rigides de liaison peuvent être en nombre supérieur à deux, en fonction du nombre d'étages instrumentés. Le dispositif peut être dépourvu, dans certains cas, d'organes intermédiaires de liaison transversale entre les tiges, cette liaison rigide étant alors assurée par les plaques terminales allongées 31 ou éventuellement 31a.

## Revendications

1. Plaque (4) pour dispositif d'ostéosynthèse de correction des déviations rachidiennes, destinée à assurer la fixation d'une seule tige (1) s'étendant sur la partie antérieure de corps vertébraux des vertèbres associées (D12-L3), des moyens étant prévus pour solidariser la plaque (4) et la tige (1), et la plaque au corps vertébral,et deux alésages (11, 12) sont percés dans la plaque pour recevoir chacun une vis (5) de fixation de la plaque au corps vertébral, ladite plaque étant incurvée et présentant une surface extérieure cintrée (7) correspondant à l'anatomie antéro-latérale des corps vertébraux,etcette plaque comporte sur sa face intérieure, opposée à la surface extérieure cintrée, un bossage (8) percé d'une ouverture (9) de passage de la tige (1).

2. Plaque selon la revendication 1, caractérisée en ce qu'au moins l'alésage (12) le plus antérieur est chanfreiné (12a) pour recevoir une vis (5) pourvue d'une tête conique (5b) épousant le chanfrein (12a), afin de permettre une orientation de la vis dans le corps vertébral (D12...).

3. Plaque selon l'une des revendications 1 et 2, caractérisée en ce que des picots sont ménagés sur sa face opposée à la surface extérieure cintrée (7) pour faciliter l'ancrage de la plaque sur la vertèbre.

4. Plaque selon l'une des revendications 1 à 3, caractérisée en ce que les alésages (11, 12) sont ménagés au voisinage des extrémités de la plaque (4) et les moyens de solidarisation de la plaque et de la tige (1) comprennent un trou taraudé (13) reliant la surface extérieure cintrée (7) au trou (9) du bossage (8), et une vis (14) adaptée pour être vissée dans ce trou taraudé (9).

5. Plaque (31) selon l'une des revendications 1 à 3, caractérisée en ce qu'elle s'étend sur une longueur suffisante pour que son alésage latéral ou postérieur (32) puisse recevoir une vis (3) de fixation de la plaque au corps vertébral et de fixation de la tige latérale ou postérieure à ladite plaque, cette vis pouvant être par exemple du type comportant un corps en U (21) et un bouchon fileté (23) de blocage de la tige (2) dans le corps, pouvant se visser à l'intérieur du corps en U sur des taraudages de ses branches latérales.

6. Plaque (31a) selon l'une des revendications 1 à 5, caractérisée en ce que l'ouverture du bossage (8) est un canal (33) ouvert sur la surface extérieure cintrée (7) pour y permettre l'insertion de la tige antérieure (1), et cette plaque est munie de moyens de blocage de la tige dans le canal, par exemple un boulon (34) pouvant se visser sur des taraudages (35) usinés sur les rives dudit canal (33).

7. Dispositif d'ostéosynthèse pour la correction de déviations rachidiennes, avec
- Deux tiges (1, 2) destinées à être fixées respectivement sur une partie antérieure et sur une partie latérale ou postérieure des corps vertébraux (D12, L1, L2, L3),
- Des moyens de fixation de la tige latérale ou postérieure (2) aux vertèbres, caractérisé en ce qu'il comprend en combinaison :
- Des plaques (4) conformes à l'une quelconque des revendications 1 à 6, dont les bossages (8) peuvent être traversés par les extrémités opposées de la tige antérieure (1),
- Des moyens (14, 8, 9) de blocage d'au moins la tige antérieure (1) dans les plaques (4) et des moyens (5) d'ancrage des plaques dans les corps vertébraux des vertèbres associées,

8. Dispositif selon la revendication 7, caractérisé en ce qu'il comprend des moyens (6) de liaison transversale rigide des deux tiges (1, 2) entre elles, constituant avec ces dernières un montage en cadre rigide.

9. Dispositif selon la revendication 7 ou 8, caractérisé en ce que les vis (5) traversant chaque plaque (4) sont munies de têtes coniques (5b) épousant les chanfreins (11a, 12a) des alésages (11, 12), afin d'éviter toute saillie de ces têtes par rapport à la surface extérieure (7) de chaque plaque (4).

10. Dispositif selon l'une des revendications 8 ou 9, caractérisé en ce que les moyens de liaison transversale rigide des tiges (1, 2) sont des barres rigides (6) équipées chacune de deux crochets terminaux (15) délimitant des gorges (16) de réception des tiges, ainsi que d'organes (18) de blocage des tiges dans les gorges des crochets.

11. Dispositif selon l'une des revendication 7 à 10, caractérisé en ce que les moyens de fixation de la tige latérale ou postérieure (2) sont des vis (3) du type comportant un corps (21) en U et un bouchon fileté (23) de blocage de la tige dans le corps, pouvant se visser à l'intérieur du corps en U sur des taraudages de ses branches latérales (21b).

12. Dispositif selon la revendication 7 ou 8, caractérisé en ce qu'il comporte à ses extrémités des plaques (31) selon la revendication 5, et entre ses extrémités des plaques (31 ou 31a ou 4) selon l'une des revendications 1, 5 et 6, assurant une liaison transversale rigide des tiges entre elles.

## Claims

1. A plate (4) for an osteosynthesis device for correcting rachidial deviations, which plate (4) is intended to ensure securing of a single rod (1) extending over the anterior part of vertebral bodies of associated vertebrae (D12 - L3), means being provided to firmly attach the plate (4) and the rod (1), and the plate to the vertebral body, and two bores (11, 12) are made in the plate in order to each receive a screw (5) for securing the plate to the vertebral body, the said plate being curved and having an arched outer surface (7) corresponding to the anterolateral anatomy of the vertebral bodies, and this plate has on its inner face - opposite the arched outer surface - a boss (8) in which there is made an opening (9) for passage of the rod (1).

2. A plate in accordance with Claim 1, characterised in that so as to enable orientation of the screw in the vertebral body (D12...), the most anterior bore (12) at least is bevelled (12a) so as to receive a screw (5) provided with a conical head (5b) corresponding to the bevel (12a).

3. A plate in accordance with any one of Claims 1 and 2, characterised in that spikes are made on the face opposite the arched outer surface (7) in order to facilitate anchoring of the plate on the vertebra.

4. A plate in accordance with any one of Claims 1 to 3, characterised in that the bores (11, 12) are made in the vicinity of the ends of the plate (4) and the means for firmly attaching the plate to the rod (1) comprise both a tapped hole (13) linking the arched outer surface (7) to the hole (9) in the boss (8) and a screw (14) adapted to be screwed into this tapped hole (9) [sic].

5. A plate (31) in accordance with any one of Claims 1 to 3, characterised in that it extends over sufficient length for its posterior or lateral bore (32) to be able to receive a screw (3) for securing the plate to the vertebral body and for securing the posterior or lateral rod to the said plate, this screw being able to be for example of the type comprising a U-shaped body (21) and a threaded plug (23) which is for locking the rod (2) in the body and can screw into the interior of the U-shaped body on internal screw threads of the lateral branches of the latter.

6. A plate (31a) in accordance with any one of Claim 1 to 5, characterised in that the opening in the boss (8) is a channel (33) open at the arched outer surface (7) so as to enable insertion of the anterior rod (1) therein, and this plate is equipped with means for locking the rod in this channel, for example a bolt (34) which can screw onto the internal screw threads (35) machined on the sides of the said channel (33).

7. An osteosynthesis device for correcting rachidial deviations, having
- two rods (1, 2) intended to be secured on an anterior part and on a posterior or lateral part of the vertebral bodies (D12, L1, L2, L3) respectively,
- means for securing the posterior or lateral rod (2) to the vertebrae, characterised in that it comprises in combination:
- plates (4) according to any one of Claims 1 to 6, through the bosses (8) of which there can pass the opposite ends of the anterior rod (1),
- means (14, 8, 9) for locking at least the anterior rod (1) in the plates (4) and means (5) for anchoring the plates in the vertebral bodies of the associated vertebrae.

8. A device in accordance with Claim 7, characterised in that it comprises means (6) for rigid transverse linking of the two rods (1, 2) to one another, constituting with the latter a rigid frame mounting.

9. A device in accordance with Claim 7 or 8, characterised in that the screws (5) passing through each plate (4) have conical heads (5b) corresponding to the bevels (11a, 12a) of the bores (11, 12), so as to prevent any projection of these heads relative to the outer surface (7) of each plate (4).

10. A device in accordance with any one of Claims 8 or 9, characterised in that the means for rigid transverse linking of the rods (1, 2) are rigid bars (6) each equipped with two terminal hooks (15) delimiting recesses (16) for receiving the rods, as well as with members (18) for locking the rods in the recesses of the hooks.

11. A device in accordance with any one of Claims 7 to 10, characterised in that the means for securing the posterior or lateral rod (2) are screws (3) of the type comprising a U-shaped body (21) and a threaded plug (23) which is for locking the rod in the body and can screw into the interior of the U-shaped body on the internal screw threads of the lateral branches (21b) of the latter.

12. A device in accordance with Claim 7 or 8, characterised in that at its ends it has plates (31) in accordance with Claim 5 and between its ends it has plates (31 or 31a or 4) in accordance with any one of Claims 1, 5 and 6 ensuring rigid transverse linking of the rods to one another.

## Patentansprüche

1. Platte (4) für eine Osteosynthesevorrichtung zur Berichtigung von Verformungen der Wirbelsäule, die dazu bestimmt ist, die Befestigung einer einzelnen Stange (1) zu gewährleisten, die sich über den vorderen Bereich von Wirbelkörpern der betreffenden Wirbel (D12-L3) erstreckt, wobei Mittel vorgesehen sind zur Verbindung der Platte (4) und der Stange (1) und der Platte mit dem Wirbelkörper, und wobei zwei Bohrungen (11, 12) die Platte durchsetzen, um jeweils eine Schraube (5) zur Befestigung der Platte am Wirbelkörper aufzunehmen, wobei diese Platte gekrümmt ist und eine gewölbte äußere Oberfläche (7) entsprechend der Anatomie des vorderen seitlichen Bereichs des Wirbelkörpers bildet und diese Platte auf ihrer der gewölbten äußeren Oberfläche entgegengesetzten Innenfläche einen Wulst (8) aufweist, der von einer Öffnung (9) für, den Durchtritt der Stange (1) durchsetzt ist.

2. Platte nach Anspruch 1, dadurch gekennzeichnet, daß wenigstens die am weitesten vorn gelegene Bohrung (12) eine Abfasung (12a) aufweist, um eine Schraube (5) aufzunehmen, die mit einem zu der Abfasung (12a) passenden konischen Kopf (5b) versehen ist, um die Ausrichtung der Schraube in dem Wirbelkörper (D12 ...) zu ermöglichen.

3. Platte nach Anspruch 1 oder 2, dadurch gekennzeichnet, daß Spitzen auf ihrer der gewölbten äußeren Oberfläche (7) entgegengesetzten Oberfläche ausgebildet sind, um die Verankerung der Platte auf dem Wirbel zu erleichtern.

4. Platte nach einem der Ansprüche 1 bis 3, dadurch gekennzeichnet, daß die Bohrungen (11, 12) in der Nähe der Enden der Platte (4) ausgebildet sind und die Mittel zur Verbindung dem Platte und der Stange (1) ein Gewindeloch (13), das die gewölbte äußere Oberfläche (7) mit dem Loch (9) des Wulstes (8) verbindet, und eine Schraube (14) umfassen, die dazu ausgebildet ist, in dieses Gewindeloch (9) eingeschraubt zu werden.

5. Platte (31) nach einem der Ansprüche 1 bis 3, dadurch gekennzeichnet, daß sie sich über eine ausreichende Länge erstreckt, damit ihre seitliche oder hintere Bohrung (32) eine Schraube (3) zur Befestigung der Platte am Wirbelkörper und zur Befestigung der seitlichen oder hinteren Stange an der Platte aufnehmen kann, wobei diese Schraube beispielsweise von der Art sein kann, die einen U-förmigen Körper (21) und einen zum Blockieren der Stange (2) in dem Körper dienenden Gewindestopfen (22) aufweist, der im Inneren dei U-förmigen Körpers mit Gewindegängen seiner seitlichen Arme in Gewindeeingriff treten kann.

6. Platte (31a) nach einem der Ansprüche 1 bis 5, dadurch gekennzeichnet, daß die Öffnung da Wulstes (8) ein an der gewölbten äußeren Oberfläche (7) offener Kanal (33) ist, um das Einsetzen der vorderen Stange (1) darin zu ermöglichen, und diese Platte mit Mitteln zum Blockieren der Stange in dem Kanal versehen ist, beispielsweise einem Bolzen (34), der mit Gewindegängen (35) in Gewindeeingriff treten kann, die in die Flanken des Kanals (33) eingearbeitet sind.

7. Osteosynthesevorrichtung zur Berichtigung von Verformungen der Wirbelsäule, mit
- zwei Stangen (1, 2), die dazu bestimmt sind, an einem vorderen Bereich bzw. einem seitlichen oder hinteren Bereich von Wirbelkörpern (D12, L1, L2, L3) befestigt zu werden,
- Mitteln zur Befestigung der seitlichen oder hinteren Stange (2) an den Wirbeln, dadurch gekennzeichnet, daß sie in Kombination aufweist:
- Platten (4) nach einem der Ansprüche 1 bis 6, deren Wülste (8) von den entgegengesetzten Enden der vorderen Stange (1) durchlaufen werden können,
- Mittel (14, 18, 19) zum Blockieren wenigstens der vorderen Stange (1) in den Platten (4) und Mittel (5) zur Verankerung der Platten in den Wirbelkörpern der betreffenden Wirbel.

8. Vorrichtung nach Anspruch 7, dadurch gekennzeichnet, daß sie Mittel (6) zur starten Querverbindung der beiden Stangen (1, 2) miteinander aufweist, die mit den letzteren ein starres Rahmengesteil bilden.

9. Vorrichtung nach Anspruch 7 oder 8, dadurch gekennzeichnet, daß die jede Platte (4) durchsetzenden Schrauben (5) mit konischen Köpfen (5b) versehen sind, die zu den Abfasungen (11a, 12a) der Bohrungen (11, 12) passen. um jegliches Vorspringen dieser Köpfe in bezug auf die äußere Oberfläche (7) jeder Platte (4) zu vermeiden.

10. Vorrichtung nach Anspruch 8 oder 9, dadurch gekennzeichnet, daß die Mittel zur starren Querverbindung der Stangen (1, 2) starre Riegel (6) sind. die jeweils mit zwei endständigen Haken (15), die Hohlkehlen (16) zur Aufnehme der Stangen begrenzen, sowie mit Organen (18) zum Blockieren der Stangen in den Hohlkehlen der Haken versehen sind.

11. Vorrichtung nach einem der Ansprüche 7 bis 10, dadurch gekennzeichnet, daß die Mittel zur Befestigung der seitlichen oder hinteren Stange (2) Schrauben (3) von der Art sind, die einen U-förmigen Körper (21) und einen zum Blockieren der Stange in dem Körper dienenden Gewindestopfen (23) aufweisen, der im Inneren des U-förmigen Körpers mit Gewindegängen seinur seitlichen Arme (21b) in Gewindeeingriff treten kann.

12. Vorrichtung nach Anspruch 7 oder 8, dadurch gekennzeichnet, daß sie an ihren Enden Platten (31) nach Anspruch 5 und zwischen ihren Enden Platten (31 oder 31a oder 4) nach einem der Ansprüche 1, 5 und 6 aufweist, die eine starre Querverbindung der Stangen miteinander gewährleisten.
